# EUROPEAN PATENT SPECIFICATION

(11) **EP 4 327 781 B1**
(45) Date of publication and mention of the grant of the patent: **03.12.2025**
(21) Application number: 22891390.1
(22) Date of filing: 16.05.2022
(51) Int. Cl.: A61F 2/24

(54) **ARTIFICIAL HEART VALVE**
KÜNSTLICHE HERZKLAPPE
VALVE CARDIAQUE ARTIFICIELLE

(30) Priority: 09.11.2021 CN 202111322399
(43) Date of publication of application: 28.02.2024
(73) Proprietor: Shanghai Trulive Medtech Co., Ltd., Shanghai 201206 (CN); Jiangsu Trulive Medtech Co., Ltd., Nantong, Jiangsu 226400 (CN)
(72) Inventor: ZHANG, Haijun, Shanghai 201206 (CN); ZHAO, Jing, Shanghai 201206 (CN); LIU, Xiang, Shanghai 201206 (CN)
(74) Representative: Pohlman, Sandra M.
(86) International application number: PCT/CN2022/092937
(87) International publication number: WO 2023/082579

(56) References cited:
- CN-A- 111 035 472
- CN-A- 111 714 250
- CN-A- 114 028 030
- CN-U- 209 091 745
- US-A1- 2016 278 923
- US-A1- 2020 100 897
- US-A1- 2021 000 592
- US-A1- 2021 220 126
- US-A1- 2021 244 535

## Description

### TECHNICAL FIELD

The present invention relates to the field of medical device and, in particular, to a prosthetic heart valve.

### BACKGROUND

Heart valves are openable and closeable flap-like structures in the organs of humans and some animals. The human heart has four valves: the aortic valve connecting the left ventricle to the aorta; the pulmonary valve connecting the right ventricle to the pulmonary artery; the mitral valve connecting the left atrium to the left ventricle; and the tricuspid valve connecting the right atrium to the right ventricle. All these valves function like one-way valves which allow blood flow in the forward direction but prevent its backward flow. With the development of social economy and the aging of population, the incidence of valvular heart disease has increased significantly. Studies have shown that this figure has reached up to 13.3% among those 75 years or older. Currently, traditional surgical treatment remains the first choice for patients with severe valvular disease. However, for those with advanced ages, complications in multiple organs, a history of thoracotomy or poor heart functions, the traditional surgical approach is associated with high risk and high mortality or even precludes some patients.

The tricuspid valve, also known as the right atrioventricular valve, has a similar structure to the mitral valve, it includes the leaflets, annulus, chordae tendineae, papillary muscles and cardiac muscles. Transcatheter tricuspid valve replacement/repair has attracted extensive attention amongst academics and researchers thanks to its advantages such as no need for thoracotomy, minor trauma and rapid recovery. Although tricuspid valve replacement technology has advanced rapidly, it is facing with a number of well-recognized challenges in terms of valve design, such as valve anchoring. Most existing tricuspid valves designs are largely oversized to provide radial support for anchoring, or employ needle-like piercing structures which hook into native tissue to form axial anchoring. Specifically, oversized anchoring refers to seating a prosthetic valve in the annulus and on tissue under the annulus. Because the radial dimension is relative large, a certain oversize is formed between the prosthetic valve and the native tissue so that a radial compression force is generated between the mesh (with diamond-shaped openings) of the prosthetic valve and the native tissue (annulus and leaflets). During cardiac cycles, the radial compression force creates axial friction therebetween that avoid relative movement between the prosthetic valve and the native tissue, thereby firmly anchoring the prosthetic valve between the right atrium and the right ventricle. In order to achieve stronger axial anchoring, some products are provided with needle-like piercing structures with a certain length to nodes of the mesh with diamond-shaped openings, which can impart greatly increased stability to axial anchoring.

However, in practical applications, such designs that utilize radial support to achieve stable axial anchoring have been found as being associated with a high risk of causing damage to native tissues such as the valve annulus, leaflets, conduction tissue and atrioventricular wall: 1) due to the radial oversize, after being implanted into the annulus, they will stretch and thus possibly damage the annulus and compress the conduction tissue or even the aorta, adversely affecting the left cardiac function; moreover, for patients with implanted permanent pacemakers (PPMs), excessive radial forces they provide may cause damage to PPM leads, or necessitate more complicated surgical procedures; and 2) the needle-like piercing structures readily tend to pierce and penetrate through the native leaflets, annulus and ventricular wall.

### SUMMARY OF THE INVENTION

It is an object of the present invention to provide a prosthetic heart valve capable of stable axial anchoring while causing minor damage to native tissue.

To this end, the present invention provides a prosthetic heart valve comprising:
an outer stent layer comprising a flange section, a main section and an attachment section sequentially joined along an axial direction thereof, wherein the flange section has a diameter gradually increasing in the axial direction away from the main section and is configured to be pressed on the tricuspid valve annulus, wherein the main section is configured to be seated inside of the tricuspid valve leaflet, wherein the attachment section comprises a septal leaflet attachment tine and an anterior and posterior leaflet attachment tine, the septal leaflet attachment tine configured to anchor to the septal leaflet of the tricuspid valve leaflet, the anterior and posterior leaflet attachment tine configured to anchor to the anterior leaflet and/or the posterior leaflet of the tricuspid valve leaflet, thereby clamping the tricuspid valve leaflet between the flange section and the attachment section, and wherein a length of the anterior and posterior leaflet attachment tine is greater length than a length of the septal leaflet attachment tine;
a skirt attached to the flange section and the main section; and
prosthetic leaflets disposed inside of the main section and configured to have an open configuration and a closed configuration.

Optionally, the length of the anterior and posterior leaflet attachment tine may lie between 4 mm and 30 mm, and the length of the septal leaflet attachment tine may lie between 2 mm and 15 mm.

Optionally, the anterior and posterior leaflet attachment tine may extend first away from an axis and then toward the flange section, and be configured to be inserted between the anterior leaflet chordae tendineae and/or between the posterior leaflet chordae tendineae, and act together with the flange section to secure the anterior leaflet and/or the posterior leaflet.

Optionally, the anterior and posterior leaflet attachment tine may comprise, a high-stiffness section and a low-stiffness section that are sequentially joined, wherein a first end of the high-stiffness section is attached to the main section, and a second end of the high-stiffness section joined is attached to the low-stiffness section, wherein the high-stiffness section extends circumferentially away from the axis and is configured to ensure a range for attachment of the anterior leaflet and/or the posterior leaflet, wherein the low-stiffness section extends axially toward the flange section, and wherein a radial gap is provided between the low-stiffness section and the main section, so that the anterior leaflet and/or the posterior leaflet can be seated between the main section and the low-stiffness section.

Optionally, the high-stiffness section may have a length lying between 2 mm and 10 mm, and the low-stiffness section may have a length lying between 5 mm and 20 mm.

Optionally, the high-stiffness section may be a metal strut, and the low-stiffness section may be a single thin strut, a braided strut or a spring strut.

Optionally, the low-stiffness section may form an angle β in the range of -15° to 15° with respect to an axis of the main section.

Additionally, the anterior and posterior leaflet attachment tine may further comprise a terminal tip and a cover layer, the terminal tip provided at an end of the low-stiffness section away from the high-stiffness section, the cover layer covering outer surfaces of the terminal tip and the low-stiffness section.

Additionally, the terminal tip may have a smooth surface, and the cover layer may be a polymeric layer.

Optionally, the septal leaflet attachment tine may extend circumferentially away from the axis around an end portion away from the flange section, and wherein the septal leaflet attachment tine is configured to: abutment against the septal leaflet or be inserted between the septal leaflet chordae tendineae; and act together with the flange section to secure the septal leaflet.

Optionally, the septal leaflet attachment tine may be a curved metal strut.

Optionally, the flange section may form an angle α in the range of -10° to 55° with respect to a radial cross-section of the main section.

Optionally, the main section may have a cylindrical shape or a conical shape and have a greater diameter than the tricuspid valve leaflet, wherein when the main section is a conical shape, its diameter gradually decreases in the axial direction away from the flange section.

Optionally, stiffness of the main section may gradually increase in the axial direction away from the flange section.

Compared with the prior art, the present invention has at least the following benefits:
In the prosthetic heart valve, the anterior and posterior leaflet attachment tine is longer than, and thus differentiated from, the septal leaflet attachment tine. This design makes it suitable for use with different anatomical configurations. The septal leaflet attachment tine is configured to anchor to the septal leaflet of the tricuspid valve leaflet, and the anterior and posterior leaflet attachment tine is configured to anchor to the anterior leaflet and/or the posterior leaflet of the tricuspid valve leaflet, thereby clamping the tricuspid valve leaflet between the flange section and the attachment section. In this way, the flange section and the attachment section of the outer stent layer can provide an axial anchoring force that is strong enough to effect axial anchoring of the prosthetic heart valve without the use of an additional needle-like piercing structure, while causing only minor damage to native tissue. Moreover, the prosthetic heart valve can be stably anchored between the right atrium and the right ventricle without requiring the main section to have a large diameter.

The documents US2021/220126 A1 and US2021/000592 A1 disclose prosthetic valves that are positioned in an annulus of a native tricuspid valve.

### BRIEF DESCRIPTION OF THE DRAWINGS

Fig. 1 is a schematic perspective diagram showing the structure of a prosthetic heart valve according to an embodiment of the present invention, which is being deployed in the heart.
Fig. 2 is a schematic perspective diagram showing the structure of a prosthetic heart valve according to an embodiment of the present invention.
Figs. 3a to 3c are simplified schematic diagrams of a prosthetic heart valve according to an embodiment of the present invention.
Figs. 4a to 4b are schematic diagrams showing the structure of septal leaflet attachment tines according to an embodiment of the present invention.
Figs. 5a to 5c are schematic diagrams showing the structure of anterior and posterior leaflet attachment tines according to an embodiment of the present invention.
Fig. 6 is a force diagram of an outer stent layer according to an embodiment of the present invention.

In these figures,
1, the right atrium; 2, the right ventricle; 3, the tricuspid valve annulus; 4, the tricuspid valve leaflet; 41, the anterior leaflet; 42, the septal leaflet; 51, the anterior leaflet chordae tendineae; 52, the septal leaflet chordae tendineae; 61, the anterior leaflet papillary muscle; 62, the septal leaflet papillary muscle;
10, a prosthetic heart valve; 100, an outer stent layer; 110, a flange section; 120, a main section; 130, an attachment section; 131, a septal leaflet attachment tine; 132, an anterior and posterior leaflet attachment tine; 1321, a high-stiffness section; 1322, a low-stiffness section; 1323, a terminal tip; 1324, a cover layer; 200, a skirt; 300, prosthetic leaflets; and 400, an inner stent layer.

### DETAILED DESCRIPTION

Prosthetic heart valves proposed in the present invention will be further described below. The present invention will be described in greater detail below with reference to the accompanying drawings, which present preferred embodiments of the invention. It would be appreciated that those skilled in the art can make changes to the invention disclosed herein while still obtaining the beneficial results thereof. Therefore, the following description shall be construed as being intended to be widely known by those skilled in the art rather than as limiting the invention.

For the sake of clarity, not all features of actual implementations are described. In the following, description and details of well-known functions and structures are omitted to avoid unnecessarily obscuring the invention. It should be appreciated that in the development of any such actual implementation, numerous implementation-specific decisions must be made to achieve specific goals of the developers, such as compliance with system-related and business-related constrains, which may vary from one implementation to another. Moreover, it should be appreciated that such a development effort might be complex and time consuming, but would nevertheless be a routine undertaking for those of ordinary skill in the art.

Objects and features of the present invention will become more apparent upon reading the following more detailed description of particular embodiments thereof reference to the accompanying drawings. Note that the figures are provided in a very simplified form not necessarily drawn to exact scale and for the only purpose of facilitating easy and clearly description of the disclosed embodiments. As used herein, the term "or" is generally employed in the sense of "and/or", unless the context clearly dictates otherwise. As used herein, the terms "inner", "outer" and similar terms are merely illustrative and do not represent the only implementation possible.

The right heart is composed of the right atrium 1, the right ventricle 2, the tricuspid valve annulus 3 at the junction of the right atrium 1 and the right ventricle 2, the tricuspid valve leaflet 4 attached thereto, papillary muscles in the right ventricle 2 and chordae tendineae thereof. A healthy heart pumps blood from the right atrium 1 into the right ventricle 2, and with the tricuspid valve leaflet 4 ensuring its one-way flow, the blood eventually flows out through the outflow tract of the right ventricle 2. When a lesion is present at the tricuspid valve, the tricuspid valve leaflet 4 may not be able to ensure complete one-way flow of blood in the heart, possibly leading to tricuspid regurgitation. The tricuspid valve leaflet 4 comprises the anterior leaflet 41, the posterior leaflet and the septal leaflet 42. The anterior leaflet chordae tendineae 51 attach at opposite ends to the anterior leaflet 41 and the anterior leaflet papillary muscle 61, and the septal leaflet chordae tendineae 52 attach at opposite ends to the septal leaflet 42 and the septal leaflet papillary muscle 62. Gaps are present at each of the adjacent septal leaflet chordae tendineae 52 and adjacent anterior leaflet chordae tendineae 51.

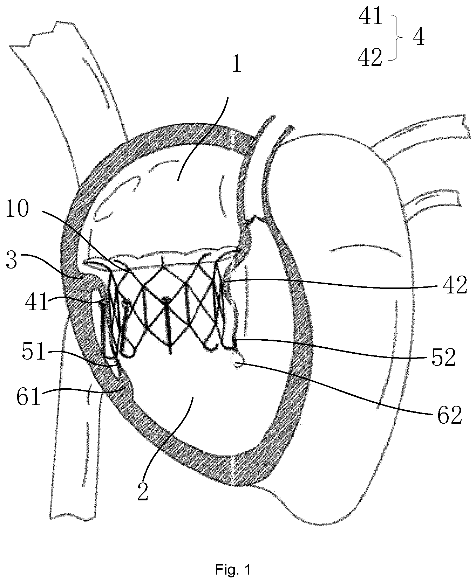

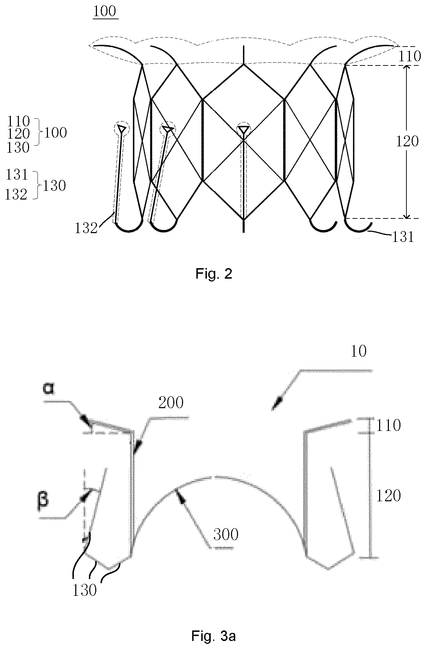

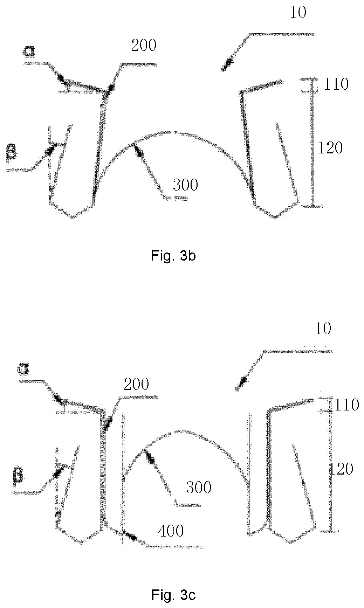

Fig. 1 is a schematic perspective diagram showing the structure of a prosthetic heart valve according to an embodiment of the present invention, which is being deployed in the heart. Fig. 2 is a schematic perspective diagram showing the structure of a prosthetic heart valve according to an embodiment of the present invention. Figs. 3a to 3c are simplified schematic diagrams of a prosthetic heart valve according to an embodiment of the present invention. As shown in Figs. 1 to 2 and 3a to 3c, in an embodiment of the present invention, a prosthetic heart valve 10 intended for transcatheter implantation into the heart and eventual anchoring between the right atrium 1 and the right ventricle 2 is provided. The prosthetic heart valve 10 can be used to treat tricuspid regurgitation by ensuring one-way flow of blood in place of the native tricuspid valve leaflet. The prosthetic heart valve 10 includes a stent, prosthetic leaflets 300 and a skirt 200. The prosthetic leaflets 300 are provided inside the stent, and the skirt 200 is attached to the stent.

Referring to Figs. 1 to 3b, the stent includes an outer stent layer 100. The prosthetic leaflets 300 are provided inside of the outer stent layer 100, and the skirt 200 is attached to the outer stent layer 100.

The outer stent layer 100 is made up of structural units axially changeable in shape, such as mesh-like, wave-shaped or other structural units. At least one row of structural units may be arranged along an axial direction of the outer stent layer, and all structural units in each row are circumferentially joined together. In case of multiple rows, the structural units in different rows may be connected directly or indirectly in the axial direction. Preferably, the mesh-like structural units may be diamond-shaped, pentagonal, hexagonal or otherwise-shaped closed structural units. The outer stent layer 100 may be a biocompatible metal frame or laser-cut solid metal tube made of, for example, nitinol, a titanium alloy, a cobalt-chromium alloy, MP35n, 316 stainless steel, L605, Phynox/Elgiloy, a platinum-chromium alloy, or another biocompatible metal. Preferably, the outer stent layer 100 is made of a shape memory alloy. The outer stent layer 100 may also be made of an elastically or plastically deformable material, such as a balloon expandable material, or a shape memory alloy which can switch, in response to temperature changes, between a collapsed configuration for delivery and an expanded or unfolded configuration. The outer stent layer 100 may also be made of braid wires or another suitable material.

The outer stent layer 100 includes a flange section 110, a main section 120 and an attachment section 130 along an axial direction thereof. The flange section 110 is flared in shape and extends along the axial direction away from the main section 120 and the attachment section 130 and is configured to cover the atrioventricular orifice (i.e., cover the tricuspid valve annulus and the inner heart wall around the tricuspid valve annulus). That is to say, the flange section 110 is an extension of the main section 120 extending from the right ventricle 2 to the right atrium 1. Moreover, a circumferential diameter of the flange section 110 gradually increases in the direction from the right ventricle 2 to the right atrium 1 so as to accommodate dimensional variation of the inner wall of the heart extending from the right atrium 2 to the tricuspid valve annulus 3. An angle α between the flange section 110 and a radial cross-section lies between -10° and 55°, and an end of the flange section 110 facing away from the main section 120 can abut against the inner wall of the heart at the tricuspid valve annulus 3. Thus, the inner wall of the heart at the tricuspid valve annulus 3 can provide the flange section 110 with axial support in the direction away from the right ventricle 2.

The main section 120 is intended to be provided in the tricuspid valve leaflet 4 and to form an interference fit with the tricuspid valve annulus 3. Additionally, a diameter of the main section 120 is slightly greater than, for example, 1-1.1 times, a diameter of the tricuspid valve annulus 3. This enables the main section 120 to exert sufficient but not excessive radial compression on the tricuspid valve annulus 3, which will not damage the tricuspid valve annulus or cause compression on the conduction tissue. Of course, in other embodiments, it is also possible for the diameter of the main section 120 to be smaller than the diameter of the tricuspid valve annulus 3. The main section 120 may be in the shape of a cylinder or a cone with a diameter decreasing from the right atrium to the right ventricle. This can facilitate stable anchoring. Stiffness of the main section 120 gradually increases in an axial direction thereof from the right atrium 1 to the right ventricle 2. Thus, a portion of the main section 120 around the right atrium is least stiff. This results in a reduced chronic outward force (COF) and less compression on surrounding tissues, such as the conduction tissue or the aorta. The main section 120 may be directly or indirectly connected to the prosthetic leaflets 300, in order to provide support to the prosthetic leaflets 300.

The attachment section 130 is an extension of the main section 120 extending from the right atrium 1 to the right ventricle 2. The attachment section 130 includes septal leaflet attachment tines 131 and anterior and posterior leaflet attachment tines 132. The anterior and posterior leaflet attachment tines 132 are configured for anchoring in the gaps between the anterior leaflet chordae tendineae 51 and/or the gaps between the posterior leaflet chordae tendineae. Specifically, the anterior and posterior leaflet attachment tines 132 are configured to be inserted into the gaps between the anterior leaflet chordae tendineae 51 and/or the gaps between the posterior leaflet chordae tendineae and thereby clamp the anterior leaflet 41 and/or the posterior leaflet between the anterior and posterior leaflet attachment tines 132 and the main section 120. Moreover, they also act together with the flange section 110 axially to clamp the anterior leaflet 41 and/or the posterior leaflet, thereby achieving stable axial anchoring of the stent and hence anchoring of the prosthetic heart valve 10. The septal leaflet attachment tines 131 are configured for anchoring on the septal leaflet or in the gaps between the septal leaflet chordae tendineae. The anterior and posterior leaflet attachment tines 132 may have a length ranging from 4 mm to 30 mm.

Anatomically, in most patients, the anterior leaflet 41 and the posterior leaflet are larger and looser than the septal leaflet, and the chordae tendineae of the septal leaflet 42 are smaller than those of the anterior leaflet 41 and the posterior leaflet. Moreover, the gaps between the septal leaflet chordae tendineae 52 are narrower and denser. Therefore, in practice, it is necessary to consider an effective leaflet attachment range of the attachment section 130 in order to ensure desired leaflet attachment efficiency and improved axial anchoring stability, while taking into account possible damage caused by the septal leaflet attachment tines 131 and the anterior and posterior leaflet attachment tines 132 to native tissue. Based on the anatomical knowledge discussed above, the anterior and posterior leaflet attachment tines 132 may be structured differently from the septal leaflet attachment tines 131 and designed with a greater length than the septal leaflet attachment tines 131.

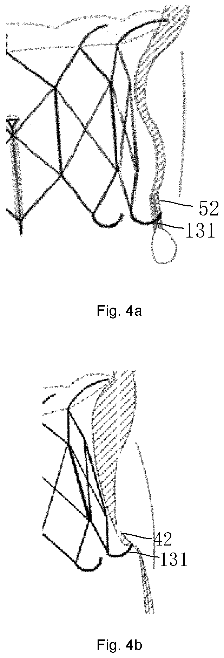

Figs. 4a to 4b are schematic diagrams showing the structure of the septal leaflet attachment tines according to an embodiment. As shown in Figs. 4a to 4b, the septal leaflet attachment tines 131 extend circumferentially away from an axis of the attachment section so as to generally comprise a shape like the letter "U". For example, the septal leaflet attachment tines 131 may be metal struts formed integrally with the main body of the stent by cutting. The septal leaflet attachment tines 131 may have a greater width and hence higher stiffness. In one embodiment, the septal leaflet attachment tines 131 may be, for example, curved metal struts. The septal leaflet attachment tines 131 may be inserted into the gaps between the septal leaflet chordae tendineae in order to secure the septal leaflet together with the flange section 110. Moreover, it may abut against the septal leaflet, in order to act together with both the interference fit of the main section 120 and the flange section 110 to secure the septal leaflet. The septal leaflet attachment tines 131 may have a shorter length, for example, in the range of 2 mm to 15 mm.

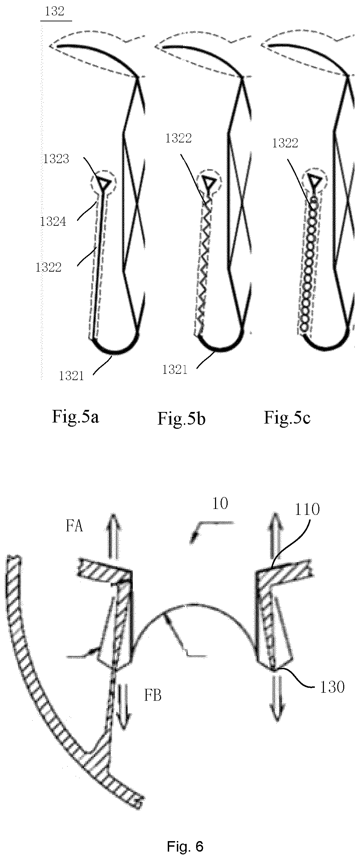

Figs. 5a to 5c are schematic diagrams showing the structure of the anterior and posterior leaflet attachment tines according to an embodiment. As shown in Figs. 5a to 5c, the anterior and posterior leaflet attachment tines 132 extend first circumferentially away from the axis and then axially toward the flange section 110. The anterior and posterior leaflet attachment tines 132 are stiffness-varying structures each including a high-stiffness section 1321 and a low-stiffness section 1322. One end of the high-stiffness section 1321 is attached to the main section 120, and the other end is joined to the low-stiffness section 1322. The high-stiffness section 1321 extends circumferentially away from the axis and is configured to pull the leaflet to form an axial anchoring force and connect the low-stiffness section, thereby ensuring a desirable anterior and/or posterior leaflet attachment range. The high-stiffness section 1321 is generally U-shaped and has a length in the range of 2 mm to 10 mm. This can facilitate stable anchoring. The high-stiffness section 1321 is a curved metal strut having a greater width than the low-stiffness section 1322, and hence it has a higher stiffness than the low-stiffness section 1322.

The low-stiffness section 1322 extends axially toward the flange section 110 and is spaced apart from the main section 120 by a radial gap. In this way, the anterior leaflet and/or the posterior leaflet can be clamped between the main section 120 and the low-stiffness section 1322. Since the low-stiffness section 1322 is less stiff, it can exert only a small radial compression force on the anterior leaflet and/or the posterior leaflet. Thus, radial compression on the anterior leaflet and/or the posterior leaflet will not be increased. The low-stiffness section 1322 is configured to expand the anterior leaflet and/or posterior leaflet attachment range and enable the anterior leaflet and/or the posterior leaflet to be clamped between the anterior and posterior leaflet attachment tines 132 and the main section 120, preventing unwanted abrupt movement of the prosthetic valve stent between the right atrium and the right ventricle and weaken the ability of the anterior and posterior leaflet attachment tines 132 to pierce and penetrate cardiac muscles.

The low-stiffness section 1322 has a length of 5 mm to 20 mm, and its stiffness is low, thereby avoiding it from being stuck to the musculi pectinati or piercing through the right ventricular wall, the tricuspid valve leaflet or the tricuspid valve annulus. Moreover, the low-stiffness section 1322 has a relatively large length, which enables it to axially restrict the prosthetic heart valve 10 over cardiac cycles, facilitating stable anchoring of the prosthetic heart valve 10. The low-stiffness section 1322 may comprise any of various forms such as a single thin strut, a braided strut, a spring strut, etc. In order to facilitate the clamping of the anterior leaflet and/or the posterior leaflet, the low-stiffness section 1322 forms an angle β in the range of -15° to 15° with an axis of the main section 120.

In one embodiment, the main section, the high-stiffness section and the low-stiffness section may be integrally formed by laser cutting. The high-stiffness section 1321 and the main section may be made up of metal struts of the same material. The low-stiffness section may be made up of laser-cut thinner metal or spring struts with a smaller width than those in the high-stiffness section 1321, or struts braided from nickel-titanium wires and with a desirable length and degree of stiffness.

The low-stiffness sections 1322 are provided at their ends away from the high-stiffness section 1321 with respective terminal tips 1323, which are fixedly attached to the low-stiffness sections 1322 by integral cutting, riveting or welding. The terminal tips 1323 are rigid and strong structures and is provided with smooth surfaces, such as spheres, rounded cones or the like, which can protect the native tricuspid valve from possible damage that can be caused by the anterior and posterior leaflet attachment tines 132. Moreover, the length of the anterior and posterior leaflet attachment tines 132 may be large enough to create a 5-9 mm axial gap between the terminal tips 1323 and the flange section. For patients with a short anterior or posterior leaflet, the terminal tips 1323 of the attachment tines may be brought into abutment with the tricuspid valve annulus from the other side (right ventricular side). This enables the stent to be axially supported at three locations in the right cardiac (the flange section on the right atrial side of the tricuspid valve annulus, the high-stiffness section at the gaps between the anterior leaflet chordae tendineae and/or the gaps between the posterior leaflet chordae tendineae, and the terminal tips 1323 on the right ventricular side of the tricuspid valve annulus), facilitating axial anchoring of the prosthetic heart valve 10 without the use of any needle-like piercing structures and allowing it to be axially restricted over cardiac cycles.

In order to avoid the anterior and posterior leaflet attachment tines 132 from causing damage to native tissue and facilitate their endothelialization, a cover layer 1324 may be provided on surfaces of the low-stiffness section 1322 and the terminal tips 1323. The cover layer 1324 may be a polymeric layer.

In embodiments of the present invention, the flange section 110, the main section 120 and the attachment section 130 may be integrally formed, or separate components which are securely joined together. The main section 120 and the attachment section 130 may be joined together by welding, suturing, gluing or otherwise, and the skirt 200 is attached to the flange section 110 and the main section 120, and the flange section 110 and the main section 120 may be securely joined through the skirt 200.

Anatomically, since the tricuspid valve annulus is relatively large, it is necessary for a portion of the outer stent layer 100 in the prosthetic valve that is intended to be implanted to replace the native tricuspid valve (i.e., the prosthetic heart valve 10 described herein) for supporting the prosthetic leaflets 300 to have a large diameter and a large axial height. This large portion of the prosthetic heart valve 10 will be located subvalvularly after the prosthetic heart valve 10 is implanted in the tricuspid valve, creating a high risk of damage to the subvalvular structure of the native valve. A double-layer stent design can delegate functions, such as supporting of the prosthetic leaflets 300, anchoring and sealing, to individual single stent layers, thereby allowing the implant to deliver its intended therapeutic effect while not affecting normal functioning of the rest of the heart. Therefore, the stent disclosed herein is preferred to be a double-layer stent.

Referring to Fig. 3c, in addition to the outer stent layer 100, the stent further includes an inner stent layer 400. The outer stent layer 100 is sleeved outside the inner stent layer 400 and comprises the same axial direction as the inner stent layer 400. The prosthetic leaflets 300 are arranged within the inner stent layer 400, and the skirt 200 is attached to the outer stent layer 100 and/or inner stent layer 400. In one embodiment, the skirt 200 is attached to both the outer stent layer 100 and inner stent layer 400 and thereby joins the outer stent layer 100 to inner stent layer 400. In other embodiments, the outer and inner stent layers may be joined otherwise, and further description thereof is omitted herein.

The inner stent layer 400 is a tubular structure providing an environment for attachment of the prosthetic leaflets 300 and the skirt 200. The inner stent layer 400 has significant radial and axial stiffness, which enables it to withstand traction from the prosthetic leaflets 300. The inner stent layer 400 is made up of structural units axially changeable in shape, such as mesh-like, wave-shaped or other structural units. At least one row is arranged along an axial direction of the inner stent layer, and all structural units in each row are circumferentially joined together. In case of multiple rows, the structural units in different rows may be connected directly or indirectly in the axial direction. The inner stent layer 400 may be made of a nickel-titanium alloy or another biocompatible material with shape memory properties, or of a cobalt-chromium alloy, stainless steel or another biocompatible material. It may be fabricated by cutting followed by heat treatment, sandblasting, polishing or other processes necessary for its fabrication. In case of the inner stent layer being fabricated from a nickel-titanium material, it may gain desired stiffness that ensures good fatigue resistance of the valve leaflets by appropriately configuring parameters including wall thickness, strut width, phase transition point, etc.

There are at least two prosthetic leaflets 300, each firmly attached to the outer stent layer 100 directly or indirectly at one end and free at the other end. In this way, the prosthetic leaflets 300 can dynamically switch between an open configuration and a closed configuration. In the closed configuration, all the prosthetic leaflets 300 sealingly abut against one another and are thereby tightly gathered or closed together. In the open configuration, a blood flow channel allowing one-way blood flow is formed between the prosthetic leaflets 300. The prosthetic leaflets 300 may be formed of any suitable material or combination of suitable materials. In particular, the prosthetic leaflets 300 may be selected from biological tissues or animal pericardial tissues, such as: chemically stabilized tissues originated from heart valves of animals (e.g., pigs), such as cattle (bovine pericardia), sheep (ovine pericardia), pigs (porcine pericardia) and horses (equine pericardia), with bovine pericardial tissues being preferred; tissues made from small intestinal submucosal tissues or made of synthetic materials, such as expanded polytetrafluoroethylenes and polyesters, particularly thermoplastic polycarbonate-urethane, polyether-urethane, segmented polyether-urethane, silicone-polyether-urethane, silicone- polycarbonate-urethane, ultra-high molecular weight polyethylene, etc; biocompatible polymers such as polyolefins, elastomers, polyethylene glycol, polyethersulfone, polysulfone, polyvinylpyrrolidone, polyvinyl chloride, other fluoropolymers, silicone polyesters, silicone polymers and/or oligomers, and/or polylactones, and block copolymers thereof. Optionally, an anticoagulant film may be provided on surfaces of the prosthetic leaflets 300. Examples of the anticoagulant may include, but are not limited to, heparinized polymers.

Fig. 6 is a force diagram of the outer stent layer according to an embodiment of the present invention. As shown in Fig. 6, during use, the flange section 110 of the outer stent layer 100 is tightly fitted and held on the tricuspid valve annulus 3 and subjected to a reaction force FA. The anterior and posterior leaflet attachment tines 132 are inserted into the gaps between the anterior leaflet chordae tendineae and/or the gaps between the posterior leaflet chordae tendineae, thereby axially clamping the anterior and posterior leaflets between the flange section 110 and the anterior and posterior leaflet attachment tines 132. The septal leaflet attachment tines 131 are brought into abutment with the septal leaflet or inserted into the gaps between the septal leaflet chordae tendineae, thereby clamping the septal leaflet between the flange section 110 and the septal leaflet attachment tines 131. The attachment section 130 is subjected to a reaction force FB. Under the action of the two forces, the outer stent layer 100 is stably anchored in the axial direction. Moreover, since the anterior and posterior leaflet attachment tines 132 are inserted in the gaps between the anterior leaflet chordae tendineae or the gaps between the posterior leaflet chordae tendineae, circumferential rotation of the outer stent layer 100 is prevented. As such, the main section 120 can firmly anchor the stent between the right atrium and the right ventricle without a large radial support force.

In summary, the present invention provides a prosthetic heart valve. By configuring different attachment tines for the anterior/posterior leaflet and for the septal leaflet, this design makes it suitable for use with different anatomical configurations. Moreover, the prosthetic valve include a flange section and an attachment section, which can provide an axial anchoring force that is strong enough to axially anchor the prosthetic heart valve. The prosthetic heart valve of the present invention entails a novel anchoring mechanism, which provides enhanced stent anchoring stability while minimizing damage to native tissue.

It is noted that, as used herein, the terms "first" and "second" are only meant to distinguish various components, elements, steps, etc. from each other rather than indicate logical or sequential orderings thereof, unless otherwise indicated or specified. In light of the above teachings, any person familiar with the art may make many possible modifications and variations to the disclosed embodiments or adapt them into equivalent embodiments, without departing from the scope of the invention. Accordingly, it is intended that any and all simple variations, equivalent changes and modifications made to the foregoing embodiments based on the substantive disclosure of the invention without departing from the scope thereof fall within this scope.

## Claims

1. A prosthetic heart valve (10) for deployment between the right atrium (1) and the right ventricle (2), comprising:
an outer stent layer (100), comprising a flange section (110), a main section (120) and an attachment section (130) sequentially joined along an axial direction thereof, wherein the flange section (110) has a diameter gradually increasing in the axial direction away from the main section (120) and is configured to be pressed on the tricuspid valve annulus (3), wherein the main section (120) is configured to be seated inside of the tricuspid valve leaflet (4), wherein the attachment section (130) comprises at least one septal leaflet attachment tine (131) and at least one anterior and posterior leaflet attachment tine (132), the septal leaflet attachment tine (131) configured to anchor to the septal leaflet (42) of the tricuspid valve leaflet (4), the anterior and posterior leaflet attachment tine (132) configured to anchor to the anterior leaflet (41) and/or the posterior leaflet of the tricuspid valve leaflet (4), thereby clamping the tricuspid valve leaflet (4) between the flange section (110) and the attachment section (130), and wherein a length of the anterior and posterior leaflet attachment tine (132) is greater than a length of the septal leaflet attachment tine (131);
a skirt (200) attached to the flange section (110) and the main section (120); and
prosthetic leaflets (300) disposed inside of the main section (120) and configured to have an open configuration and a closed configuration.

2. The prosthetic heart valve (10) of claim 1, wherein the length of the anterior and posterior leaflet attachment tine (132) ranges from 4 mm to 30 mm, and wherein the length of the septal leaflet attachment tine (131) ranges from 2 mm to 15 mm.

3. The prosthetic heart valve (10) of claim 1, wherein the anterior and posterior leaflet attachment tine (132) extends first away from an axis and then toward the flange section (110), and wherein the anterior and posterior leaflet attachment tine (132) is configured to: be inserted between anterior leaflet chordae tendineae (51) and/or between posterior leaflet chordae tendineae; and act together with the flange section (110) to secure the anterior leaflet (41) and/or the posterior leaflet.

4. The prosthetic heart valve (10) of claim 3, wherein the anterior and posterior leaflet attachment tine (132) comprises a high-stiffness section (1321) and a low-stiffness section (1322) that are sequentially joined, wherein a first end of the high-stiffness section (1321) is attached to the main section (120), and a second end of the high-stiffness section (1321) is attached to the low-stiffness section (1322), wherein the high-stiffness section (1321) has a higher stiffness than the low-stiffness section (1322), wherein the high-stiffness section (1321) extends circumferentially away from the axis and is configured to ensure a range for attachment of the anterior leaflet (41) and/or the posterior leaflet, wherein the low-stiffness section (1322) extends axially toward the flange section (110), and wherein a radial gap is provided between the low-stiffness section (1322) and the main section (120), so that the anterior leaflet (41) and/or the posterior leaflet can be seated between the main section (110) and the low-stiffness section (1322).

5. The prosthetic heart valve (10) of claim 4, wherein the high-stiffness section (1321) comprises a length ranging from 2 mm to 10 mm and the low-stiffness section (1322) comprises a length ranging from 5 mm to 20 mm.

6. The prosthetic heart valve (10) of claim 4, wherein the high-stiffness section (1321) is a metal strut and the low-stiffness section (1322) is a single thin strut, a braided strut or a spring strut.

7. The prosthetic heart valve (10) of claim 4, wherein the low-stiffness section (1322) forms an angle β in a range of -15° to 15° with respect to an axis of the main section (120).

8. The prosthetic heart valve (10) of claim 4, wherein the anterior and posterior leaflet attachment tine (132) further comprises a terminal tip (1323) and a cover layer (1324), wherein the terminal tip (1323) is provided at an end of the low-stiffness section (1322) away from the high-stiffness section (1321), and wherein the cover layer (1324) covers outer surfaces of the terminal tip (1323) and the low-stiffness section (1322).

9. The prosthetic heart valve (10) of claim 8, wherein the terminal tip (1323) has a smooth surface and the cover layer (1324) is a polymeric layer.

10. The prosthetic heart valve (10) of claim 1, wherein the septal leaflet attachment tine (131) extends circumferentially away from an axis around an end away from the flange section (110), and wherein the septal leaflet attachment tine (131) is configured to: abut against the septal leaflet (42) or be inserted between the septal leaflet chordae tendineae (52); and act together with the flange section (110) to secure the septal leaflet (42).

11. The prosthetic heart valve (10) of claim 10, wherein the septal leaflet attachment tine (131) is a curved metal strut.

12. The prosthetic heart valve (10) of claim 1, wherein the flange section (110) forms an angle α in a range of -10° to 55° with respect to a radial cross-section of the main section (120).

13. The prosthetic heart valve (10) of claim 1, wherein the main section (120) comprises a cylindrical shape or a conical shape and has a greater diameter than the tricuspid valve leaflet (4), wherein when the main section (120) comprises the conical shape, the diameter of the main section (120) gradually decreases in the axial direction away from the flange section (110).

14. The prosthetic heart valve (10) of claim 1, wherein a stiffness of the main section (120) gradually increases in the axial direction away from the flange section (110).

## Patentansprüche

1. Prothetische Herzklappe (10) zum Einsatz zwischen dem rechten Vorhof (1) und der rechten Herzkammer (2), umfassend:
eine äußere Stentschicht (100), die einen Flanschabschnitt (110), einen Hauptabschnitt (120) und einen Befestigungsabschnitt (130) umfasst, die entlang ihrer Axialrichtung nacheinander verknüpft sind, wobei der Flanschabschnitt (110) einen Durchmesser aufweist, der in der Axialrichtung vom Hauptabschnitt (120) weg allmählich zunimmt, und konfiguriert ist, um auf den Trikuspidalklappenring (3) gedrückt zu werden, wobei der Hauptabschnitt (120) konfiguriert ist, um im Inneren des Trikuspidalklappensegels (4) zu sitzen, wobei der Befestigungsabschnitt (130) mindestens eine Befestigungszinke (131) für das septale Segel und mindestens eine Befestigungszinke (132) für das vordere und hintere Segel umfasst, wobei die Befestigungszinke (131) für das septale Segel konfiguriert ist, um am septalen Segel (42) des Trikuspidalklappensegels (4) verankert zu werden, und die Befestigungszinke (132) für das vordere und hintere Segel konfiguriert ist, um am vorderen Segel (41) und/oder dem hinteren Segel des Trikuspidalklappensegels (4) verankert zu werden, wodurch das Trikuspidalklappensegel (4) zwischen dem Flanschabschnitt (110) und dem Befestigungsabschnitt (130) geklemmt wird, und wobei eine Länge der Befestigungszinke (132) des vorderen und hinteren Segels größer ist als eine Länge der Befestigungszinke (131) für das septale Segel;
eine Schürze (200), die am Flanschabschnitt (110) und dem Hauptabschnitt (120) befestigt ist; und
prothetische Segel (300), die im Inneren des Hauptabschnitts (120) angeordnet sind und konfiguriert sind, um eine offene und eine geschlossene Konfiguration aufzuweisen.

2. Prothetische Herzklappe (10) nach Anspruch 1, wobei die Länge der Befestigungszinke (132) des vorderen und hinteren Segels im Bereich von 4 mm bis 30 mm liegt, und wobei die Länge der Befestigungszinke (131) für das septale Segel im Bereich von 2 mm bis 15 mm liegt.

3. Prothetische Herzklappe (10) nach Anspruch 1, wobei sich die Befestigungszinke (132) des vorderen und hinteren Segels zunächst von einer Achse weg und dann in Richtung des Flanschabschnitts (110) erstreckt, und wobei die Befestigungszinke (132) des vorderen und hinteren Segels konfiguriert ist, um: zwischen die Chordae tendineae des vorderen Klappensegels (51) und/oder zwischen die hinteren Sehnenfäden des Segels eingesetzt zu werden; und mit dem Flanschabschnitt (110) zusammenzuwirken, um das vordere Segel (41) und/oder das hintere Segel zu sichern.

4. Prothetische Herzklappe (10) nach Anspruch 3, wobei die Befestigungszinke (132) des vorderen und hinteren Segels einen Abschnitt mit hoher Steifigkeit (1321) und einen Abschnitt mit geringer Steifigkeit (1322) umfasst, die nacheinander verknüpft sind, wobei ein erstes Ende des Abschnitts mit hoher Steifigkeit (1321) am Hauptabschnitt (120) befestigt ist und ein zweites Ende des Abschnitts mit hoher Steifigkeit (1321) am Abschnitt mit geringer Steifigkeit (1322) befestigt ist, wobei der Abschnitt mit hoher Steifigkeit (1321) eine höhere Steifigkeit aufweist als der Abschnitt mit geringer Steifigkeit (1322), wobei sich der Abschnitt mit hoher Steifigkeit (1321) in Umfangsrichtung von der Achse weg erstreckt und konfiguriert ist, um einen Bereich zur Befestigung des vorderen Segels (41) und/oder des hinteren Segels zu gewährleisten, wobei sich der Abschnitt mit geringer Steifigkeit (1322) axial in Richtung des Flanschabschnitts (110) erstreckt und wobei zwischen dem Abschnitt mit geringer Steifigkeit (1322) und dem Hauptabschnitt (120) ein radialer Spalt bereitgestellt ist, sodass das vordere Segel (41) und/oder das hintere Segel zwischen dem Hauptabschnitt (110) und dem Abschnitt mit geringer Steifigkeit (1322) platziert werden kann.

5. Prothetische Herzklappe (10) nach Anspruch 4, wobei der Abschnitt mit hoher Steifigkeit (1321) eine Länge im Bereich von 2 mm bis 10 mm umfasst und der Abschnitt mit geringer Steifigkeit (1322) eine Länge im Bereich von 5 mm bis 20 mm umfasst.

6. Prothetische Herzklappe (10) nach Anspruch 4, wobei der Abschnitt mit hoher Steifigkeit (1321) eine Metallstrebe ist und der Abschnitt mit geringer Steifigkeit (1322) eine einzelne dünne Strebe, eine geflochtene Strebe oder eine Federstrebe ist.

7. Prothetische Herzklappe (10) nach Anspruch 4, wobei der Abschnitt mit geringer Steifigkeit (1322) einen Winkel β in einem Bereich von -15° bis 15° in Bezug auf eine Achse des Hauptabschnitts (120) bildet.

8. Prothetische Herzklappe (10) nach Anspruch 4, wobei die Befestigungszinke (132) des vorderen und hinteren Segels weiter eine Kontaktspitze (1323) und eine Abdeckschicht (1324) umfasst, wobei die Kontaktspitze (1323) an einem Ende des Abschnitts mit geringer Steifigkeit (1322) vom Abschnitt mit hoher Steifigkeit (1321) weg bereitgestellt ist und wobei die Abdeckschicht (1324) die Außenoberflächen der Kontaktspitze (1323) und den Abschnitt mit geringer Steifigkeit (1322) bedeckt.

9. Prothetische Herzklappe (10) nach Anspruch 8, wobei die Kontaktspitze (1323) eine glatte Oberfläche aufweist und die Abdeckschicht (1324) eine Polymerschicht ist.

10. Prothetische Herzklappe (10) nach Anspruch 1, wobei sich die Befestigungszinke (131) für das septale Segel von einer Achse weg um ein vom Flanschabschnitt (110) entferntes Ende herum umfänglich erstreckt und wobei die Befestigungszinke (131) für das septale Segel konfiguriert ist, um: an dem septalen Segel (42) anzuliegen oder zwischen die Chordae tendineae (52) des septalen Segels eingesetzt zu werden; und mit dem Flanschabschnitt (110) zusammenwirkt, um das septale Segel (42) zu sichern.

11. Prothetische Herzklappe (10) nach Anspruch 10, wobei die Befestigungszinke (131) für das septale Segel eine gekrümmte Metallstrebe ist.

12. Prothetische Herzklappe (10) nach Anspruch 1, wobei der Flanschabschnitt (110) einen Winkel α in einem Bereich von -10° bis 55° in Bezug zu einem radialen Querschnitt des Hauptabschnitts (120) bildet.

13. Prothetische Herzklappe (10) nach Anspruch 1, wobei der Hauptabschnitt (120) eine zylindrische Form oder konische Form umfasst und einen größeren Durchmesser als das Trikuspidalklappensegel (4) aufweist, wobei, wenn der Hauptabschnitt (120) die konische Form umfasst, der Durchmesser des Hauptabschnitts (120) in der Axialrichtung vom Flanschabschnitt (110) weg allmählich abnimmt.

14. Prothetische Herzklappe (10) nach Anspruch 1, wobei die Steifigkeit des Hauptabschnitts (120) in der Axialrichtung vom Flanschabschnitt (110) weg allmählich zunimmt.

## Revendications

1. Valve cardiaque prothétique (10) destinée à être déployée entre l'oreillette droite (1) et le ventricule droit (2), comprenant :
une couche de stent externe (100), comprenant une section de rebord (110), une section principale (120) et une section de fixation (130) reliées séquentiellement le long d'une direction axiale de celles-ci, dans laquelle la section de rebord (110) présente un diamètre augmentant progressivement dans la direction axiale en s'éloignant de la section principale (120) et est configurée pour être pressée sur l'anneau de valve tricuspide (3), dans laquelle la section principale (120) est configurée pour être placée à l'intérieur du feuillet de valve tricuspide (4), dans laquelle la section de fixation (130) comprend au moins une dent de fixation de feuillet septal (131) et au moins une dent de fixation de feuillets antérieur et postérieur (132), la dent de fixation de feuillet septal (131) étant configurée pour s'ancrer au feuillet septal (42) du feuillet de valve tricuspide (4), la dent de fixation de feuillets antérieur et postérieur (132) étant configurée pour s'ancrer au feuillet antérieur (41) et/ou au feuillet postérieur du feuillet de valve tricuspide (4), serrant ainsi le feuillet de valve tricuspide (4) entre la section de rebord (110) et la section de fixation (130), et dans laquelle une longueur de la dent de fixation de feuillets antérieur et postérieur (132) est supérieure à une longueur de la dent de fixation de feuillet septal (131) ;
une jupe (200) fixée à la section de rebord (110) et à la section principale (120) ; et
des feuillets prothétiques (300) disposés à l'intérieur de la section principale (120) et configurés pour présenter une configuration ouverte et une configuration fermée.

2. Valve cardiaque prothétique (10) selon la revendication 1, dans laquelle la longueur de la dent de fixation de feuillets antérieur et postérieur (132) va de 4 mm à 30 mm, et dans laquelle la longueur de la dent de fixation de feuillet septal (131) va de 2 mm à 15 mm.

3. Valve cardiaque prothétique (10) selon la revendication 1, dans laquelle la dent de fixation de feuillets antérieur et postérieur (132) s'étend d'abord à l'écart d'un axe, puis vers la section de rebord (110), et dans laquelle la dent de fixation de feuillets antérieur et postérieur (132) est configurée pour : être insérée entre les cordages tendineux de feuillet antérieur (51) et/ou entre les cordages tendineux de feuillet postérieur; et agir conjointement avec la section de rebord (110) pour fixer le feuillet antérieur (41) et/ou le feuillet postérieur.

4. Valve cardiaque prothétique (10) selon la revendication 3, dans laquelle la dent de fixation de feuillets antérieur et postérieur (132) comprend une section à rigidité élevée (1321) et une section à rigidité faible (1322) qui sont jointes séquentiellement, dans laquelle une première extrémité de la section à rigidité élevée (1321) est fixée à la section principale (120), et une seconde extrémité de la section à rigidité élevée (1321) est fixée à la section à rigidité faible (1322), dans laquelle la section à rigidité élevée (1321) présente une rigidité supérieure à celle de la section à rigidité faible (1322), dans laquelle la section à rigidité élevée (1321) s'étend circonférentiellement à l'écart de l'axe et est configurée pour assurer une plage de fixation du feuillet antérieur (41) et/ou du feuillet postérieur, dans laquelle la section à rigidité faible (1322) s'étend axialement vers la section de rebord (110), et dans laquelle un espace radial est prévu entre la section à rigidité faible (1322) et la section principale (120), de sorte que le feuillet antérieur (41) et/ou le feuillet postérieur peuvent être placés entre la section principale (110) et la section à rigidité faible (1322).

5. Valve cardiaque prothétique (10) selon la revendication 4, dans laquelle la section à rigidité élevée (1321) comprend une longueur allant de 2 mm à 10 mm et la section à rigidité faible (1322) comprend une longueur allant de 5 mm à 20 mm.

6. Valve cardiaque prothétique (10) selon la revendication 4, dans laquelle la section à rigidité élevée (1321) est une entretoise métallique et la section à rigidité faible (1322) est une entretoise mince simple, une entretoise tressée ou une entretoise à ressort.

7. Valve cardiaque prothétique (10) selon la revendication 4, dans laquelle la section à rigidité faible (1322) forme un angle β dans une plage de -15° à 15° par rapport à un axe de la section principale (120).

8. Valve cardiaque prothétique (10) selon la revendication 4, dans laquelle la dent de fixation de feuillets antérieur et postérieur (132) comprend en outre une pointe terminale (1323) et une couche de recouvrement (1324), dans laquelle la pointe terminale (1323) est prévue à une extrémité de la section à rigidité faible (1322) à l'écart de la section à rigidité élevée (1321), et dans laquelle la couche de recouvrement (1324) recouvre les surfaces extérieures de la pointe terminale (1323) et de la section à rigidité faible (1322).

9. Valve cardiaque prothétique (10) selon la revendication 8, dans laquelle la pointe terminale (1323) présente une surface lisse et la couche de recouvrement (1324) est une couche polymère.

10. Valve cardiaque prothétique (10) selon la revendication 1, dans laquelle la dent de fixation de feuillet septal (131) s'étend circonférentiellement à l'écart d'un axe autour d'une extrémité éloignée de la section de rebord (110), et dans laquelle la dent de fixation de feuillet septal (131) est configurée pour : venir en butée contre le feuillet septal (42) ou être insérée entre les cordages tendineux de feuillet septal (52) ; et agir conjointement avec la section de rebord (110) pour fixer le feuillet septal (42).

11. Valve cardiaque prothétique (10) selon la revendication 10, dans laquelle la dent de fixation de feuillet septal (131) est une entretoise métallique incurvée.

12. Valve cardiaque prothétique (10) selon la revendication 1, dans laquelle la section de rebord (110) forme un angle α dans une plage de -10° à 55° par rapport à une section transversale radiale de la section principale (120).

13. Valve cardiaque prothétique (10) selon la revendication 1, dans laquelle la section principale (120) comprend une forme cylindrique ou une forme conique et présente un diamètre supérieur à celui du feuillet de valve tricuspide (4), dans laquelle lorsque la section principale (120) comprend la forme conique, le diamètre de la section principale (120) diminue progressivement dans la direction axiale en s'éloignant de la section de rebord (110).

14. Valve cardiaque prothétique (10) selon la revendication 1, dans laquelle une rigidité de la section principale (120) augmente progressivement dans la direction axiale en s'éloignant de la section de rebord (110).
